# EUROPEAN PATENT APPLICATION

(11) **EP 0 933 635 A2**
(43) Date of publication of application: **04.08.1999**
(21) Application number: 98124205.0
(22) Date of filing: 17.12.1998
(51) Int. Cl.: G01N 33/18

(54) **Microelectronics probe for automatic, simultaneous control and monitoring of several chemical physical parameters of liquid substances, and their transmission to remote stations**

(30) Priority: 17.12.1997 IT GE970106
(71) Applicant: Gazzari, Anna, 17015 Celle Ligure (IT)
(72) Inventor: Gazzari, Anna, 17015 Celle Ligure (Savona) (IT); Luschi, Stefano, 57100 Livorno (IT)
(74) Representative: Chirone, Giuseppe

(57) **Abstract**

This invention relates to electronic devices, which are capable to continuously measure, through a planned sampling, some chemical-physical parameters of liquid substances - in particular, water and water solutions - and record and transmit such data without any human intervention. These parameters include (but are not limited to): temperature, relative hydrostatic pressure as level indicator, ionic conductivity, pH and redox potential, concentration of specific ions (Cl⁻, F⁻, O⁻⁻, NH₄⁺, NO₃⁻⁻, metal ions, etc.). This invention is characterised in the fact that all the sensing elements are implemented on a single substrate and all the electronics performing such functions is hosted in the same housing of the sensor. Most of the unique probe functions are implemented by the hardware and software combination, and the probe itself can be described as an integrated system. This results in a very compact device with low power consumption, easy installation and maintenance and a lower overall operating cost.

## Description

### 1. Prior Art.

In general, this invention relates to electronic devices, which are capable to continuously measure, through a planned sampling, some chemical-physical parameters of liquid substances - in particular, water and water solutions - and record and transmit such data without any human intervention. These parameters include (but are not limited to): temperature. relative hydrostatic pressure as a level indicator, ionic conductivity, pH and Redox potential, concentration of specific ions (Cl⁻, F⁻, O⁻⁻, NH₄⁺, NO₃⁻⁻, metal ions, etc.).

Typical applications of such devices - possibly organised as nodes in a control network - include:
Monitoring and control of groundwater:
Aqueduct drinking waters;
Surface waters (streams, rivers, lakes, and sea);
Watershed, adjacent to waste treatment or industrial plants:
Swimming pool waters;
Aquaculture plant waters;
Aquarium waters;
Agriculture irrigation and rice cultivation waters;
Waste waters from industrial plants or zootechny.

Process control in industrial plants. More specifically:
- chemical industry
- wood processing and paper fabrication industry
- tanning industry
- Pharmaceutical and bioengineering. More specifically: process control for fermentation processes, i.e. breweries and synthesis of pharmaceutically active substances.
- Food processing, especially in dairies.

Usually measurement and monitoring functions are implemented by means of separate sensors, which generate analog voltage or current signals, one for each measured property.

All systems used to measure Redox potentials, pH or ionic concentrations require many elements, consisting of glass vials and ampoules with saline solutions. Although these elements are miniaturised, they are rather bulky. References indicate that individual electrodes or arrays of electrodes can be manufactured by using special inks and thick-film technology on ceramic substrates (Thick Film Sensors/ Edited by M. Prudenziati - 1994, ELSEVIER SCIENCE, pages 319 to 337)

Almost all these sensors have a common feature, i.e. the signal, which is generated in analogical form (current or voltage), must be transmitted, to a separate electronic unit by a cable for signal processing and displaying. This is a serious problem for high impedance potentiometric measurements and whenever long cables are required, such as in the case of underground or surface waters. In fact, a long cable acts as an antenna and this may affect measurement quality, due to noise and electromagnetic interference.

As far as multi-parameter probes are concerned, currently available products feature separate sensing elements assembled inside an individual housing which therefore is rather bulky, they require a separate unit for data processing and storage (data logger). In case of permanent installations, these units must be installed and suitably protected.

### 2.Description of the invention.

The object of this invention overcomes the above mentioned limitations. In particular, it exhibits the following unique set of features:

The instrument:
- can automatically perform its calibration procedures.
- can simultaneously measure several parameters, and record time, date and a confidence indicator for each parameter reading.
- can automatically check the electrode equilibrium status during measurement of electrochemical potentials or related quantities, and record this information.
- can accept power supply voltages in a broad range, in order to facilitate its field use even with batteries and solar cell panels.
- can be operated for a long time even in case of power supply interruptions or failures, thanks to its rechargeable battery-operated buffer system.
- can retain any already acquired and stored data whenever a power supply failure occurs, even when the battery goes low.
- can minimise power absorption. therefore it can be operated for a long time without an external power source.
- is protected against electrostatic discharges.
- can respond to specific events detected during measurement and start predefined measurement sequences and/or send alarm signals to the remote station.
- can be remotely programmed, setting up measurement frequency and sequence, alarm levels. if any, and/or sending alarm signals as described above;
- communicates with the outer world via a relatively noise-free, serial digital connection;
- can be configured in such a way to operate in a data acquisition network.

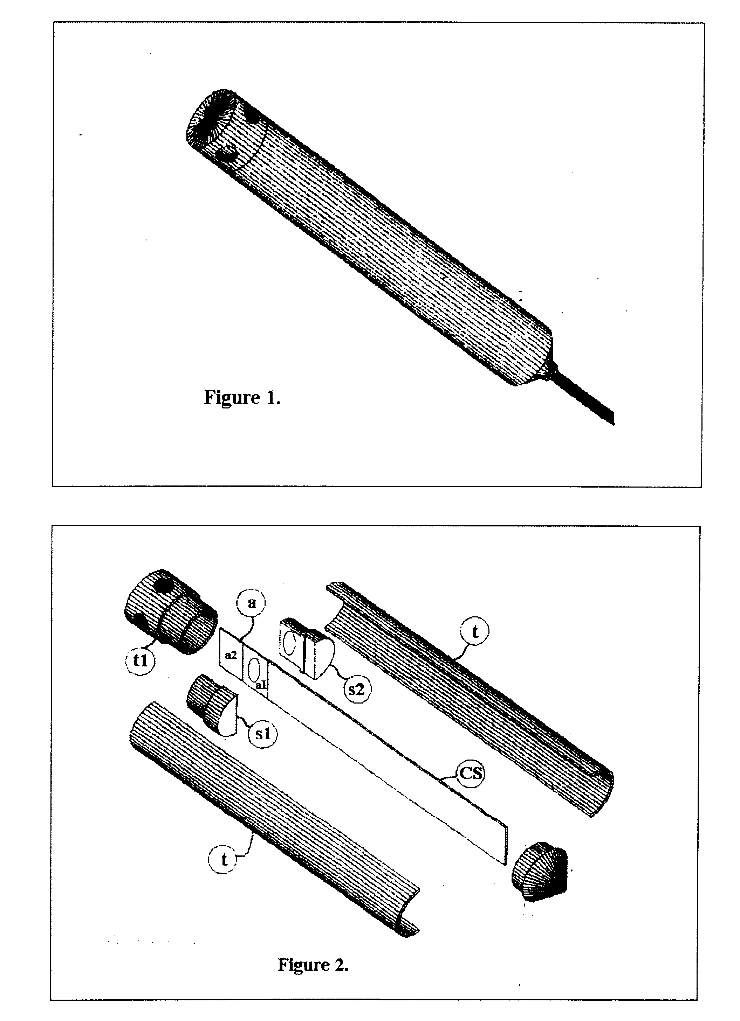

An example of this invention is illustrated in Figures 1 & 2 (exploded view).

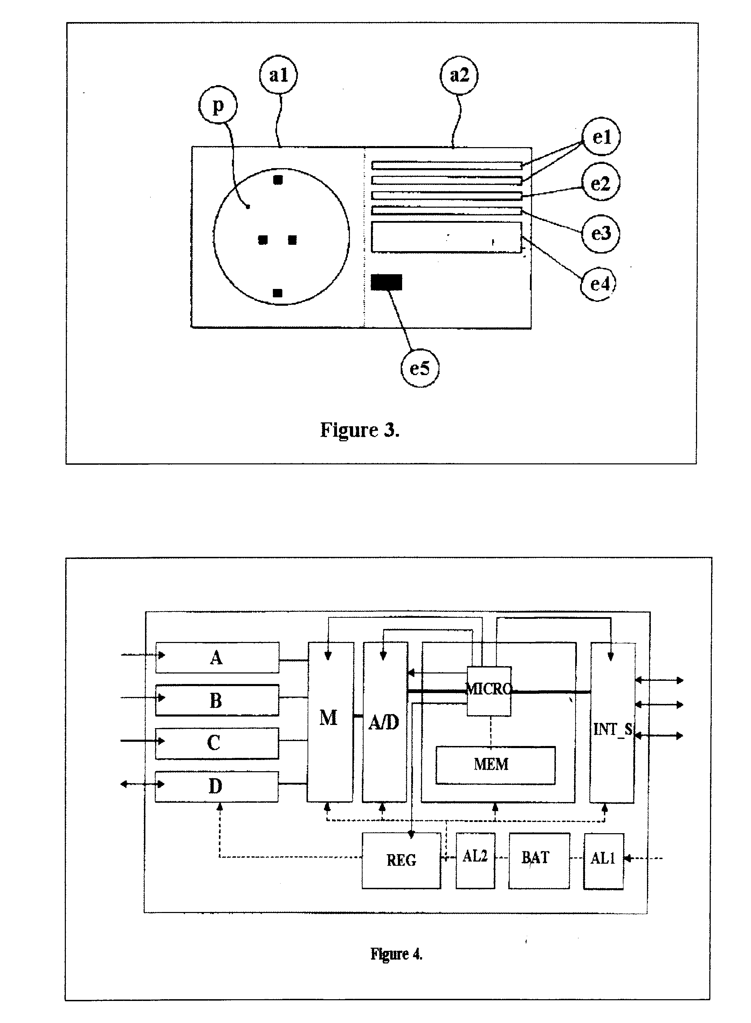

The multi-parameter probe consists of a ceramic element, Figure 2 (a), or similar, where all required sensors are implemented by an individual thick film technology process. More specifically, the invention refers to a ceramic substrate, or a similar material substrate, which includes 2 sections, Figure 3. Section (a1) contains a pressure sensor (p), consisting of a piezoresistive resistor bridge onto a ceramic membrane, or a similar material membrane. Section (a2) contains several sensing elements, manufactured by thick film technology:
- Platinum electrodes, or other electrochemically inert material electrodes, for water conductivity measurement, Figure 3 (e1). In contrast with facing electrodes described in prior art, coplanar electrodes remarkably facilitate production process, cut production costs, and simplify holder design. Any reading non-linear behaviour arising from the specific geometry used can be compensated at software level in the signal processing electronics.
- Platinum electrode, or other electrochemically inert material (or standard) electrode. again manufactured by thick film technology, Figure 3 (e2), for Redox potential measurement with respect to a reference electrode.
- Platinum electrodes, or other electrochemically inert material (or standard) electrodes, coated with a membrane, pervious to specific ions, for example ion H+, Figure 3 (e3). This permeable layer may consist of polymers, resins, or mixtures, as described in the literature (Thick Film Sensors/ Edited by M. Prudenziati - 1994, ELSEVIER SCIENCE, pages 348 to 350). An alternative known method for the manufacturing of membranes permeable to specific ions uses special glasses, again applied by thick film technologies. Both types of electrodes can be used for pH measurement or, more generally, for the measurement of a potential related to a specific ion concentration, by measuring the equilibrium potential with respect to a reference electrode.
- a reference electrode or zero electrode, Figure 3 (e4), can also be obtained on the same substrate by thick film technology. The manufactured prototypes have a Silver electrode, whose surface is converted into AgCl by an anodic oxidation process in a Cl⁻-ion containing solution. Once modified, this electrode is coated with a special layer, consisting of a fine AgCl suspension bonded with a polymeric binder, permeable to Ag+ ions.
- Resistive element for temperature measurement, obtained by, for example, platinum resistors, or resistors made by other PTC (Positive Temperature Coefficient) or NTC (Negative Temperature Coefficient) materials, provided that they can be applied by a thick film technology process, Figure 3 (e5).

Depending on the specific measurement needs, one can implement all the above mentioned elements. or a portion of them. The implementation of many measurement functions onto the same ceramic substrate offers remarkable advantages in comparison with prior art in terms of device size and simplification of the assembling process.

In the shown example, and with no exclusion of alternative housing solutions, a couple of semicylindrical elements, Figure 2 (S1 and S2), clamps the section (a1) of the ceramic substrate (a), thus forming a cylindrical structure that fits into the tubular element of the probe (t), leaving the section (a2) of the above ceramic substrate protruding off the cylinder itself. A third cylindrical element, shaped as a cup, is suitably positioned in order to protect the sensing elements, and presents holes in order to allow the above elements to get in contact with the solution to be measured (t1).

The couple of semicylindrical elements, Figure 2 (S1 and S2) are designed to seal the membrane of the pressure sensor. These elements are drilled in such a way that the opposite sides of the membrane are put in contact with the liquid to be measured, and the atmospheric reference pressure, respectively.

The probe electronics is assembled onto a printed wiring board, Figure 2 (CS), electrically connected to the sensing elements on the ceramic substrate, and can be housed in the same enclosure.

Most of the unique probe functions are implemented by the hardware and software combination, and the probe itself can be described as an integrated system. The PWB, Figure 2 (CS), is schematically depicted in Figure 4: an analogic section of the circuit is made of several independent channels (A,B,C,D,...), one for each quantity to measure. The power supply section of the circuit (AL1, BAT, AL2, REG) is designed to provide a very stable voltage to each analogical channel, in order to prevent the measurement accuracy from being affected by unexpected changes of the external power load.

As an example, assuming that the properties to be measured are: Level, Temperature, Conductivity and Redox Potential, we might configure the system in the following manner:
- Channel (A) is connected to the piezoresistive resistor bridge which measures the relative pressure on the membrane of the ceramic element, as described above.
- Channel (B) measures the temperature by a bridge circuit in which an arm is provided by the thick film resistor with positive or negative temperature coefficient (PTC or NTC), as described in the previous paragraphs.
- Channel (C) measures the conductivity linked to the electrical impedance between two thick film platinum electrodes manufactured as described above. This measurement is preferably performed with a sinusoidal voltage, at a frequency of 1 kHz and stabilised in amplitude, applied to a resistive bridge in which an arm is provided by the electrodes mentioned above.
- Channel (D) provides the measure of pH or Redox potential by measuring the potential attained at the equilibrium by the corresponding thick film electrodes on the ceramic substrate with reference to the Ag/AgCl electrode potential as described above.

On the same board, the several signals are suitably amplified in voltage and are sent to a multiplexing circuit, Figure 4 (M), that switches the signals over the input of an analog/digital converter of suitable accuracy (A/D). This converter is suitably driven by a microcontroller (MICRO) that also manages all the other functions of the probe. In fact, the microcontroller in following stage of the board processes the digital signals according to a specific program coded into the memory of the microcontroller itself. The functions performed by the circuit include the capability to provide a clock signal. The data once processed, are stored into an electronic memory, Figure 4 (MEM), together with time and date in which the measurement was performed.
Furthermore, the microcontroller drives a serial interface circuit (INT_S), again located on the board. With no exclusion of alternative solutions, this interface can be, in a specific application, of the so-called "standard RS232" type, driven by a suitable bydirectional communication software. This interface allows the probe to exchange data and programs with external units.

Depending on the specific application for which these probes are designed, the power supply section is designed to operate with a broad range of voltages starting from 1 volt. As example, it can operate in the range between 4.5 and 25 Volts. In this way, the probe can be easily powered with batteries or solar cell panels in remote stations.
Furthermore, a rechargeable backup battery assures continuous operation even in the case the external power is interrupted for a long time. Power consumption is further reduced as the probe is designed to automatically disconnect the analogical sections when no readings are programmed. In this condition, only the microcontroller and the communication interface are powered, with a remarkable reduction of the overall power consumption.

The manufactured prototypes can operate with no external power for several hours.

The probe is provided with readout software program running on a conventional personal computer which provides the following functions (in combination with the operating system in the microcontroller memory and the specific data allocation of the probe memory):
- a parameter measurement procedure consisting of a fast sequence of single sampling readings (for example, 256) followed by the calculation of average values and other statistical parameters, and the storage in the probe memory of these data, together with time and date of the measurement.
- an internal program for automatic probe calibration. involving the read-out of the parameters of well known reference solutions and the storage of these data, together with the expected values, in a specific section of the memory. In this way, during the normal operation, the readout program is enabled to recover these data for the automatic correction of the readings via software.
- a program designed to set up alarm limits in order to enable the probe to automatically react to specific events, performing predefined readings, or sending alarm signals to a remote station.
- a program designed to remotely set up time and frequency of the readings.
- a program designed to identify the probe and his technical characteristics when the probe is operated as a node in a data acquisition network where many probes can be used.
- a program designed to verify the equilibrium condition for the electrodes and to set up a suitable warm-up time before starting the measurements.

## Claims

1. A probe for water monitoring and control, in which several sensing elements coexist on a single substrate, thus allowing to simultaneously measure heterogeneous chemical or physical properties.

2. A probe as claimed in Claim 1, in which the sensing elements are implemented by thick film techniques on a ceramic substrate, or similar material.

3. A probe as claimed in one or more previous claims, in which one of the quantities to be measured is hydrostatic pressure, and this parameter is obtained by a thick film resistor bridge, whereas resistors have piezoresistive features and are deposited onto a membrane on the ceramic substrate; whereas the membrane in question bends under the pressure exerted by the liquid.

4. A probe as claimed in one or more previous claims, in which one of the quantities to be measured is the liquid temperature, and this parameter is obtained by a thick film resistor bridge with a positive or negative temperature coefficient (PTC or NTC), deposited onto the above ceramic substrate, or a similar material.

5. A probe as claimed in one or more previous claims, in which one of the quantities to be measured is the liquid conductivity, and this parameter is obtained by conductance measurements, at a frequency in the 10 Hz to 10 GHz range, between coplanar thick film electrodes deposited on the same substrate and implemented with a noble metal such as platinum or gold.

6. A probe as claimed in one or more previous claims, in which one of the quantities to be measured is the Redox potential of the liquid, and this parameter is obtained by reading the equilibrium potential between coplanar thick film electrodes deposited on the same substrate, whereas one of them (the measuring electrode) is made of a noble metal layer, such as platinum or gold, and the other is a suitable reference electrode.

7. A probe as claimed in one or more previous claims, in which one of the quantities to be measured is the pH of the liquid, and this parameter is obtained by reading the equilibrium potential between coplanar thick film electrodes deposited on the same substrate, whereas one of them (the measuring electrode) is made of a noble metal layer, such as platinum or gold, coated with a organic or inorganic film, such as glass, selectively permeable to the hydrogen ion, and the other is a suitable reference electrode.

8. A probe as claimed in one or more previous claims, in which the several analog signals are processed, converted into digital signals, stored and sent, simultaneously or after a delay time, via a serial interface; whereas these functions are performed by a microprocessor electronic circuit, loaded with a predefined operating system, and directly connected to the ceramic substrate which contains the sensing elements, or implemented in thick film technology on the same substrate, in both cases housed by the same probe enclosure.

9. A probe as claimed in one or more previous claims, in which the electronic circuit features an analog section made up of several detection and amplification channels, each of them being interfaced with one analog-to-digital converter via a switching circuit driven by the microprocessor, where the supply voltage is stabilised at a level lower than the logic's section by a voltage regulator able to switch power off when requested by the microprocessor.

10. A probe as claimed in one or more previous claims, in which the power supply circuit consists of:
• a step-down switching circuit stage, where the external supply voltage is reduced from any value in a broad range, including, but not limited to, the 6 to 25 Volts range, down to a fixed value, including, but not limited to, 4.5 Volts)
• a second stage, consisting of a rechargeable battery and current-limiting circuit serially connected to the above circuit, which can be recharged by the previous stage and provide, even in case of a external power failure, a voltage ranging from 1 Volt to the battery voltage (including, but not limited to, the 1 to 4.5 Volts range).
• a step-up switching circuit stage, able to pick up the current from the previous stage at a voltage in the predefined range (including, but not limited to, the 1 to 4.5 Volts range) and raise it up to a fixed value, such as 5 Volts, which is fed to the rest of the circuit.

11. A probe as claimed in one or more previous claims, in which the microprocessor can be so programmed as to bring the rest of the circuit, except the serial interface, in a standby condition, thus minimising power consumption.
